# EUROPEAN PATENT APPLICATION

(11) **EP 3 575 320 A1**
(43) Date of publication of application: **04.12.2019**
(21) Application number: 18174760.1
(22) Date of filing: 29.05.2018
(51) Int. Cl.: C07K 16/28, A61K 35/17, C12N 5/0783, A61K 35/12, A61K 39/00

(54) **NEW THERAPY FOR TREATING GRAFT-VERSUS-HOST-DISEASE**

(71) Applicant: Heinrich-Pette-Institut Leibniz-Institut für Experimentelle Virologie, 20251 Hamburg (DE)
(72) Inventor: NIEHRS, Annika, 22850 Norderstedt (DE); ALTFELD, Marcus, 20251 Hamburg (DE); GARCIA BELTAN, Wilfredo F., 20251 Hamburg (DE)
(74) Representative: Uexküll & Stolberg

(57) **Abstract**

The present invention relates to new compounds that are effective in treating or preventing Graft-versus-Host disease (GVHD) in a subject. More specifically, the present invention relates to an anti-NKp44 antibody or a fragment thereof for use in a method of treating or preventing GVHD in a subject. The invention also relates to a pharmaceutical composition comprising an anti-NKp44 antibody or a fragment thereof for use in a method of treating or preventing GVHD in a subject. The anti-NKp44 antibody or fragment thereof can be administered in combination with other active ingredients which are routinely used for treating or preventing GVHD. The present invention further relates to a kit comprising, as a first component, an anti-NKp44 antibody or a fragment thereof and, as a second component, an immunosuppressant.

## Description

### NEW THERAPY FOR TREATING GRAFT VERSUS HOST DISEASE

The present invention relates to new compounds that are effective in treating or preventing Graft-versus-Host disease (GVHD) in a subject. More specifically, the present invention relates to an anti-NKp44 antibody or a fragment thereof for use in a method of treating or preventing GVHD in a subject. The invention also relates to a pharmaceutical composition comprising an anti-NKp44 antibody or a fragment thereof for use in a method of treating or preventing GVHD in a subject. The anti-NKp44 antibody or fragment thereof can be administered in combination with other active ingredients which are routinely used for treating or preventing GVHD. The present invention further relates to a kit comprising, as a first component, an anti-NKp44 antibody or a fragment thereof and, as a second component, an immunosuppressant.

### BACKGROUND

Acute graft-versus-host disease (GVHD) is a common complication of allogeneic hematopoietic stem cell transplant (HSCT) that often occurs in the early post-transplantation period. It is thought to be primarily a T cell mediated disease that occurs when immune cells transplanted from a non-identical donor recognize the transplant recipient as foreign, thereby initiating an immune reaction that causes disease in the transplant recipient. The skin, gastrointestinal tract, and liver are the principal target organs in patients with acute GVHD.

Clinically significant acute GVHD occurs in 10-50 percent of patients who receive an allogeneic HSCT from a genotypically HLA-identical sibling, despite intensive prophylaxis with immunosuppressive agents, such as methotrexate, cyclosporine, or corticosteroids. Acute GVHD is more common following HCT from matched unrelated donors and haploidentical donors than in fully HLA-matched donors.

Current methods to prevent and treat GVHD have included general immune suppression following transplantation, reduced intensity conditioning, and depletion or inhibition of alloreactive donor T lymphocytes prior to transfusion. The clinical effectiveness of these methods, however, is limited by a variety of side effects. For example, general immune suppression leads to an increased risk of reactivation of latent virus infections and opportunistic infections, while reduced intensity conditioning regimens are associated with increased relapse rates. Currently, the most promising prophylactic treatment for GVHD is depletion or inhibition of donor T lymphocytes. This can be accomplished through a variety of methods including lymphoablative cytotoxic agents, specific T lymphocyte inhibitors, and T cell depletion by selecting for CD34+ hematopoietic stem and progenitor cells. While these methods are effective at lowering the rates of GVHD, they are also associated with an increased risk for life-threatening infections.

Major histocompatibility antigens or Human Leukocyte Antigens (HLA) are encoded by genes in the major histocompatibility complex (MHC). Minor histocompatibility antigens are products of protein degradation presented by HLA molecules at the cell surface of Antigen Presenting Cells (APCs). Polymorphic HLA mismatches between the donor and the recipient cells are a significant risk factor for GVHD development. For this reason, when possible, donor and recipient pairs will be HLA-matched. The matching is done according to the major antigens. Despite these precautions, GVHD can still occur within HLA-matched pairs.

It has been described that the cell surface receptor HLA-DP plays an important role in the development of GVHD. HLA-DP is a peptide-antigen receptor that is composed of the two subunits DPα and DPβ, each of which is encoded by a separate gene locus in the MHC Class II region on human chromosome 6. HLA-DP functions as a cell surface receptor for antigens taken up from the extracellular space. The immune system surveys antigens for foreign pathogens when presented by different MHC receptors, such as HLA-DP. The MHC Class II antigens are found on antigen presenting cells (APCs), such as macrophages, dendritic cells, and B-lymphocytes. In the particular context of GVHD, it was found that high expression of HLA-DP is associated with a high risk of GVHD (Petersdorf et al. (2015), New Engl. J. Med., 373, 7 pages 599-609.)

Accordingly, there is a need for novel compounds that are effective in treating and preventing GVHD and which are not associated with the disadvantages indicated above. It is an object of the invention to provide new therapeutic compositions and regimens for treating and preventing GVHD that are more effective and/or better tolerated than currently available treatment options.

It has now been surprisingly found that the cell surface receptor NKp44, which occurs on natural killer (NK) cells, binds to HLA-DP, but not to HLA-DR or HLA-DQ. It was known that the activity of NK cells is tightly regulated by an array of activating and inhibitory receptors. While interactions between NK-cell receptors and HLA class I molecules are well established, such interactions have not been described with HLA class II molecules like HLA-DP. The insight that HLA-DP molecules interact with NKp44 can be used for treating or preventing GVHD by inhibiting the interaction between the two molecules through blocking of NKp44. According to the invention, blocking of NKp44 can be effected by a molecule that specifically binds to NKp44, such as an antibody or an antibody fragment.

### BRIEF DESCRIPTION OF THE DRAWINGS

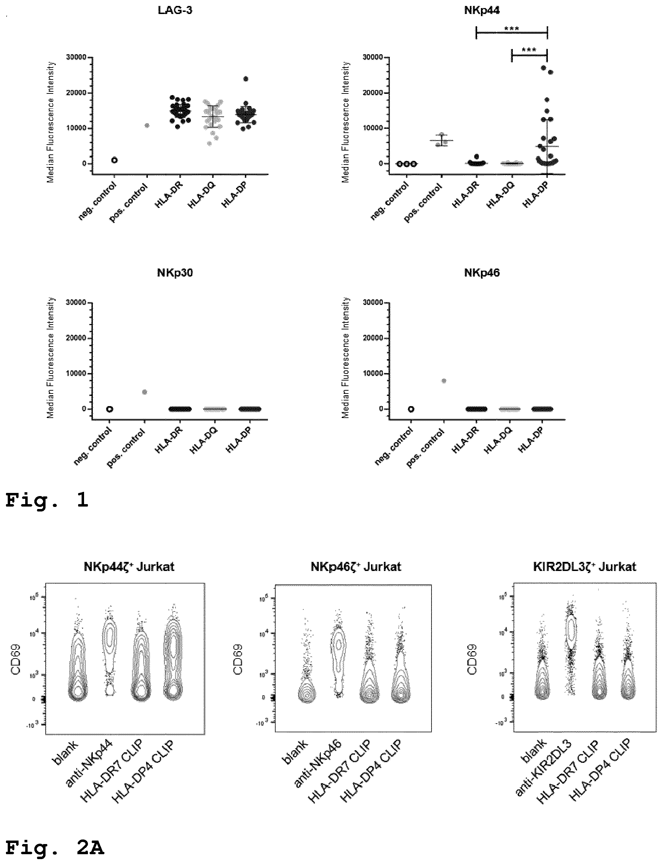

Figure 1 shows the results from analysing the potential binding of Lag-3 and NCR-Fc constructs to HLA class II coated beads. Fc-construct binding to HLA class II coated beads is plotted as median fluorescence intensity. All Fc-constructs were used at a final concentration of 30pg/mL. Each dot indicates an individual HLA class II allele. Nkp44-Fc construct binding to HLA class II beads was determined in three independent experiments. Line determines the mean. Error bars depict the standard error of the mean.

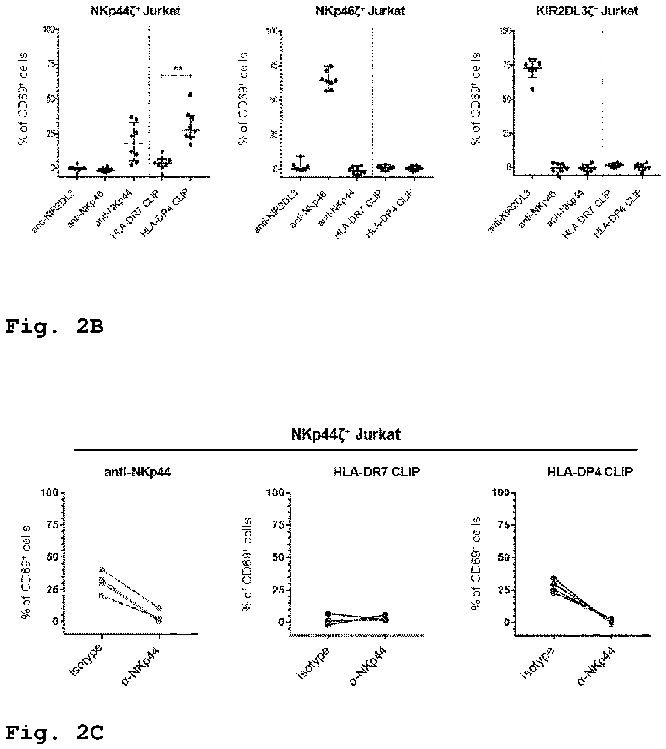

Figure 2 shows the reporter cell activity against plate-coated HLA-DR7 and HLA-DP4 monomers. A: Reporter cell activity of NKp44ζ+, NKp46ζ+, KIR2DL3ζ+ Jurkat reporter cells was determined by percentage of CD69+ cells after co-incubation with plate-coated anti-KIR2DL3, anti-NKp46, anti-NKp44, HLA-DR7 monomers loaded with CLIP peptide, HLA-DP4 monomers loaded with CLIP peptide and non-coated wells (blank). Plots are representative for eight independent experiments. B: Percentage of CD69+ Jurkat reporter cells is depicted after co-incubation with the indicated antibodies and monomers. Percentages shown are subtracted from percentage of CD69+ cells incubated on non-coated wells (blank). Values are illustrated as median with interquartile range. Data is representative for eight individual experiments. Each dot represents one technical replicate. ** p=0.0078. C: Reporter cell activity of NKp44ζ+ Jurkat cells in response to anti-NKp44, HLA-DR7 and HLA-DP4 CLIP monomers was determined in the presence of purified mouse IgG1 isotype and purified anti-human NKp44 antibody (both at a final concentration of 10pg/mL) and is depicted as percentage of CD69+ cells. All values shown are subtracted from the percentage of CD69+ NKp44ζ+ Jurkat cells incubated on non-coated wells. Data is representative of four independent experiments. The median with interquartile range is indicated in the first and fourth column of each plot. Each dot represents one individual experiment. Each line connects functional responses after incubation with the same monomer.

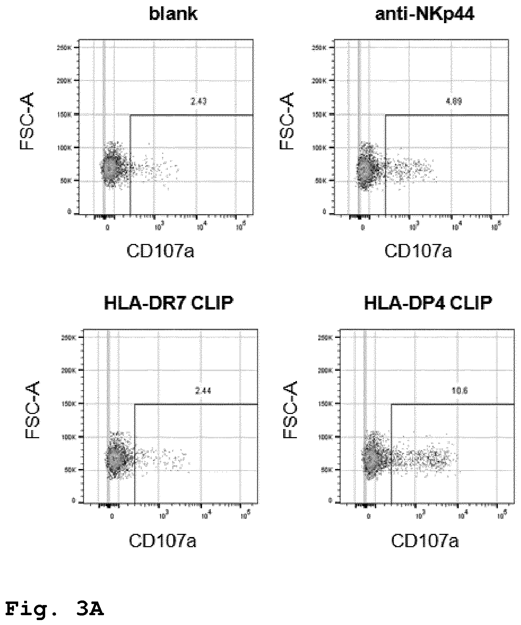

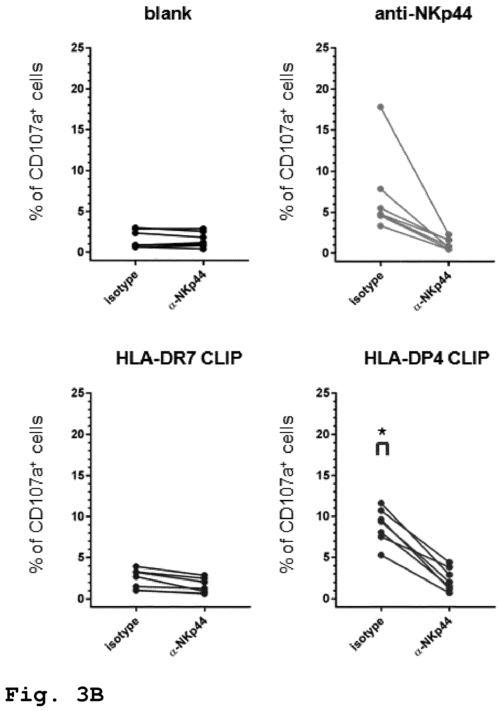

Figure 3 shows the results from the degranulation experiments. A: Percentage of CD107a+ NK cells was determined after co-incubation with the indicated plate-coated ligands or non-coated wells (blank). Plots are representative for six independent experiments. B: Degranulation after co-incubation with anti-NKp44 antibody, HLA-DR7 monomers and HLA-DP4 monomers loaded with CLIP peptide and non-coated wells (blank) is depicted as percentage of CD107a+ NK cells. Frequency of CD107a+ NK cells was determined in the presence of purified mouse IgG1 isotype and purified anti-human NKp44 antibody (both at a final concentration of 10pg/mL). Each dot represents an individual donor. Each line connects responses from individual donors. Data is representative for seven independent experiments. *indicates the difference between the response of NK cells to HLA-DR7 CLIP and HLA-DP401 when pre-incubated with purified mouse IgG1 isotype control antibody. *p=0.0313.

### DETAILED DESCRIPTION OF THE INVENTION

Accordingly, in a first aspect, the present invention provides an anti-NKp44 antibody or a fragment thereof for use in a method of treating or preventing Graft-versus-Host disease (GVHD) in a subject.

The subject to be treated according to the invention can be any mammalian that express NKp44 including, but not limited to, humans and non-human primates. In a preferred embodiment, the subject is a human. In a preferred embodiment, the subject suffers from GVHD or is at risk of developing GVHD, e.g. a subject that is subjected to hematopoietic cell transplantation.

As used herein, an "anti-NKp44 antibody" encompasses any antibody that specifically recognizes the NKp44 receptor and has the capacity to block or significantly reduce NKp44-mediated signalling. Preferably, NKp44-mediated signal transduction induced by binding of the HLA-DP ligand is inhibited by the anti-NKp44 antibody or antibody fragment by at least 50%, 60%, 70%, 80%, 90%, 95%, 96%, 97%, 98%, 99%, or even more preferably 100%. The antibodies used within the scope of the invention may be any type of antibody, e.g., polyclonal, monoclonal, chimeric, humanized, synthetic or anti-idiotypic antibodies. Suitable anti-NKp44 antibodies can belong to any of the five different classes of antibodies, i.e. IgG, IgA, IgM, IgD and IgE. Preferably, the antibodies for use in the therapeutic method of the invention are derived from the class IgG, more preferably from the sub-classes IgG1, IgG2, IgG2a, IgG2b or IgG3. The antibodies can be obtained from any mammal that has been described in the art in connection with the production of antibodies, e.g., rabbits, rats, mice, goats, horses, primates or humans. Alternatively, the antibodies may be synthetic antibodies which can be produced, for example, by recombinant processes.

According to a preferred aspect, the anti-NKp44 antibody is a monoclonal antibody. As used herein, a "monoclonal antibody" refers to an immunoglobulin which is produced by a cell line which is based on a single B lymphocyte. A monoclonal anti-NKp44 antibody is directed to a single epitope of the NKp44 antigen. The production of monoclonal antibodies based on hybridoma cell lines has been extensively described in the art (Köhler and Milstein, Nature, 256, 495-397, (1975); Harlow and Lane "Antibodies": Laboratory Manual, Cold Spring Harbor Laboratories (1988); Ausubel et al., (ed.), 1998, Current Protocols in Molecular Biology, John Wiley & Sons, New York). Apart from methods based on hydridoma cells, other methods for the production of monoclonal antibodies have been described. These methods are equally suitable for the production of antibodies for use in the context of the invention. For example, monoclonal antibodies can be produced by recombinant DNA processes as described, e.g. in US Patent 4,816,567. Monoclonal antibodies may also be isolated from an antibody phage library such as described by Clackson et al. (1991), Nature, 352:624-628. Based on the comprehensive literature available, the skilled person will have no problems preparing an anti-NKp44 antibody for therapeutic use.

When the subject to be treated with the antibody is a human, the anti-NKp44 antibody to be used is preferably humanized. Humanized anti-NKp44 antibodies comprise only a minimal sequence which is of non-human origin. The major part of the sequence of a humanized antibody is derived from a human immunoglobulin sequence. Normally, humanized antibodies are produced based on human immunoglobulins in which one or more amino acids from the complementarity-determining region (CDR) are replaced by corresponding amino acids of a non-human antibody having the desired binding characteristics. Methods for producing humanized antibodies have been described in the prior art and are known to the skilled person (see, e.g. Jones et al. (1986) Nature 321: 522-525; Verhoeyen et al. (1988) Science 239: 1534-1536). The humanized antibodies contemplated by the invention can also comprise additional amino acids which are not present in the recipient antibody or in the donor antibody. These modifications may become desirable to modify (i.e. increase or decrease) the affinity of the humanized antibody. In a preferred embodiment, the humanized antibody comprises hypervariable regions from a non-human immunoglobulin, while all or substantially all of the framework regions are derived from a human sequence.

NKp44-binding fragments of an anti-NKp44 antibody may also be used in the methods of the invention as long as these fragments specifically bind to NKp44 and have the capability to block or significantly reduce NKp44-mediated signalling. An antigen-binding fragment of an anti-NKp44 antibody may be a Fv, Fab, F(ab') and F(ab')2 fragment. Further included are single chain Fv antibody fragments and disulfide-linked Fv fragments (dsFv). Single chain antibody fragments (scFv) comprise the variable domain of the heavy chain (VH) and variable region of the light chain (VL) of an antibody, wherein these regions are linked by a peptide bridge or by disulfide bond. Methods for recombinantly producing antibody fragments have been described in the prior art and comprise, for example, techniques such as phage display or ribosome display. The recombinant antibody fragments produced by these methods may be purified and tested for binding affinity to NKp44.

An anti-NKp44 antibody of the invention or a fragment thereof exhibits a binding affinity for NKp44 which is higher than that for other cell-surface proteins. Preferably, the anti-NKp44 antibody of the present invention will have a binding affinity for NKp44 which is at least 2, 5, 10, 15, 20, 25, 50, or at least 100 times higher than its binding affinity for any other cell surface receptor protein.

The present therapy is based on the concurrent administration of a therapeutically effective amount of an anti-NKp44 antibody or antibody fragment. A therapeutically effective amount of the anti-NKp44 antibody or antibody fragment refers to an amount that, when administered to the subject, results in a therapeutic response with respect to the disease to be treated, i.e. GVHD. A therapeutically effective amount of the anti-NKp44 antibody or antibody fragment in particular causes a reduction in at least one of the typical signs of GVHD, such as damage to the liver, skin, mucosa, or gastrointestinal tract. A therapeutically effective amount of the anti-NKp44 antibody or antibody fragment may also reduce GVHD-related inflammation, sloughing of the mucosal membrane, diarrhea, abdominal pain, nausea, and vomiting. The therapeutically effective amount of the anti-NKp44 antibody or antibody fragment will depend on several parameters, such as the mode of administration, the particular type of the GVHD to be treated, the severity of the disease, the history of the disease, the age, height, weight, health, and physical condition of the subject to be treated, and the like.

A therapeutically effective amount of the anti-NKp44 antibody or antibody fragment can be determined by one of ordinary skill in the art without undue experimentation given the disclosure set forth herein. The antibody will preferably be administered to a subject in an amount that is sufficient to achieve a plasma concentration from about 0.05 pg/ml to about 150 pg/ml, preferably from about 0.1 to about 50 pg/ml, more preferably from about 1 pg/ml to about 20 pg/ml, and usually from about 1 pg/ml to about 10 pg/ml, e.g. 5 pg/ml.

In other words, the weekly dose can vary from about 1 mg to about 500 mg antibody per m² of the subject body surface. Preferably, the weekly dose of the anti-NKp44 antibody will be from about 10-300 mg/m², more preferably from 100-200 mg/m², most preferably 150 mg/m². Where the anti-NKp44 antibody is a fragment of an anti-NKp44 antibody, the amount to be administered can readily be adjusted based on the mass of the fragment relative to the mass of the whole antibody. The anti-NKp44 antibody of the invention can be administered via several different routes (see below). Preferably, the anti-NKp44 antibody is administered parenterally by injection or infusion.

In a preferred aspect, the anti-NKp44 antibody is a monoclonal antibody. In another preferred aspect, anti-NKp44 antibody is a humanized antibody. A number of different anti-NKp44 antibodies have been described in the prior art which are suitable for being used in the methods of the invention. For example, a monoclonal mouse anti-NKp44 IgG2b antibody (17-3369-42) can be purchased from ThermoFisher Scientific (Schwerte, Germany). Another monoclonal mouse anti-NKp44 IgG2b antibody (1G6) can be purchased from Novus Biologicals (Wiesbaden, Germany). Another monoclonal mouse anti-NKp44 IgG2b antibody (clone 253415) is available from R&D Systems (Wiesbaden, Germany). Another monoclonal mouse anti-NKp44 IgG1 antibody (clone P44-8) is available from BioLegend (Koblenz, Germany).

The anti-NKp44 antibody may be formulated for being delivered by different routes of administration, for example, orally in the form of tablets, capsules, granule, powder, liquids, and the like. It is however preferred that the anti-NKp44 antibody or a fragment of such an antibody is administered parenterally by intravenous injection or intravenous infusion. Administration by intravenous infusion, e.g. short-term infusion within less than 60 min, e.g. within 30 min, 20 min or 15 min, is particularly preferred.

The anti-NKp44 antibody or a fragment thereof can be used for treating acute and chronic GVDH. In a preferred embodiment, the GVDH to be treated is acute GVDH, i.e. GVHH that occurs within 100 days from transplantation. In another preferred embodiment, the GVDH to be treated is chronic GVDH, i.e. the disease form that manifests more than hundred days after transplantation.

The antibodies or antibody fragments contemplated for treating GVHD can be administered in combination with other active ingredients that are routinely used for treating or ameliorating GVHD. Specifically, the anti-NKp44 antibody or antibody fragment can be administered in combination with an immunosuppressant. As used herein, the term "immunosuppressant" refers to compounds, such as small molecules, which partially or completely inhibit the function of the immune system. In a preferred embodiment, the immunosuppressant is a glucocorticoid, calcineurin inhibitor or mTOR inhibitor.

Glucocorticoids suppress cell-mediated immunity by inhibiting cytokine production, e.g. the production of IL-1, IL-2, and TNF-alpha. A reduced cytokine production effectively results in a reduced T cell proliferation. Glucocorticoids also suppress the humoral immunity by B cell clone expansion and antibody synthesis. Preferred glucocorticoids include prednisone, dexamethasone, and hydrocortisone. Calcineurin inhibitors are compounds which inhibit the activity of calcineurin, an enzyme that activates T-cells. Preferred calcineurin inhibitors for use according to the invention include, but are not limited to, tacrolimus and cyclosporine. The primary target of sirolimus, everolimus and zotarolimus is the cell-cycle regulatory protein mTOR. The inhibition of mTOR results in a suppression of cytokine-driven T-lymphocyte proliferation.

Again, the specific amount of the immunosuppressant which is administered to the subject in combination with the anti-NKp44 antibody will depend on parameters, such as the mode of administration, the specific type of immunosuppressant, the type of GVHD disease, age, height, weight, health, and physical condition of the subject to be treated, and others. A therapeutically effective amount of an immunosuppressant is typically such that when administered is sufficient to achieve a plasma concentration of from about 0.05 pg/ml to about 250 pg/ml, preferably from about 0.1 pg/ml to 200 pg/ml, more preferably from about 1 pg/ml to about 100 pg/ml, and usually from about 10 pg/ml to about 50 pg/ml, e.g. 25 pg/ml. Stated differently, the weekly dose can vary from about 1-100 mg/m², more preferably from 10-50 mg/m², most preferably 20-25 mg/m². The weekly dose is administered in one or more distinct administrations.

The immunosuppressants may be administered in any suitable form that does not interfere with its pharmacological activity. For example, the immunosuppressants may be administered orally in the form of tablets, capsules, granule, powder, liquids, and the like. Alternatively, the immunosuppressants may be formulated for being administered by intravenous injection or intravenous infusion. In a preferred aspect, the immunosuppressant is administered to the subject to be treated by intravenous infusion, more preferably by short-term infusion within less than 60 min, e.g. within 30 min, 20 min or 15 min.

Compositions suitable for injection and/or infusion include solutions or dispersions and powders for the extemporaneous preparation of injectable solutions or dispersions. The composition for injection must be sterile and should be stable under the conditions of manufacturing and storage. Preferably, the compositions for injection and/or infusion also include a preservative, such as chlorobutanol, phenol, ascorbic acid, thimerosal, and the like. For intravenous administration, suitable carriers may comprise physiological saline, bacteriostatic water, Cremophor EL™ (BASF) or phosphate buffered saline (PBS). Sterile solutions for injection and/or infusion can be prepared by incorporating the active ingredient (the antibody and/or the immunosuppressant) in the required amount in an appropriate solvent followed by filter sterilization.

Where the anti-NKp44 antibody is administered in combination with an immunosuppressant, the anti-NKp44 antibody and the immunosuppressant can be administered to the subject in the form of a single pharmaceutical composition comprising both therapeutic agents and pharmaceutically acceptable excipients and carriers. Administration of such a pharmaceutical composition will result in a simultaneous delivery of the anti-NKp44 antibody and the immunosuppressant. Alternatively, the two therapeutic agents may also be administered separately from each other, i.e. in the form of two separate pharmaceutical compositions, one containing the antibody, and the other containing the immunosuppressant. The two separate compositions can be administered simultaneously, i.e. at the same time at two distinct sites of administration, or they may be administered sequentially (in either order) to the same site or to different sites of administration.

Preferably, both the composition comprising the anti-NKp44 antibody and the composition comprising the immunosuppressant are administered according to a weekly dosing regimen, more preferably a regimen in which a single dose of the antibody and a single dose of the immunosuppressant are administered every week for a treatment period of 2 or more weeks, for example, 3, 4, 5, 6, 7, 8, 9, 10, 11, 12, 13, 14, 15, 16, 17, 18, 19, 20, or more weeks. Preferably, the treatment period comprises at least 12 weeks. It will also be possible to administer the total weekly dose of the antibody and/or the immunosuppressant in more than one administration per week, e.g. in 2 or 3 administrations per week. In a preferred embodiment, the amount of the antibody to be administered weekly is delivered as a single intravenous infusion per week, and the amount of the immunosuppressant to be administered weekly is also given as a single intravenous infusion per week, either at the same day of administration of the antibody, e.g. within about 10 minutes to about 6 hours after administration of the antibody has been completed, more preferably within about 30, 60, 90, 120, 150, 180, 210 or 240 minutes, or at any of days 2, 3, 4, 5, 6 or 7 of the week.

For example, a combination therapy with the above-mentioned agents that is based on a weekly dosing regimen begins on day 1 of a treatment period, and a first therapeutically effective dose of the anti-NKp44 antibody is administered on that day. A first therapeutically effective dose of an immunosuppressant can be administered either on the same day, e.g. simultaneously or within about 30, 60, 90, 120, 150, 180, 210 or 240 minutes after administration of the antibody. Alternatively, the immunosuppressant can be administered at any of days 2, 3, 4, 5, 6 or 7 of the first week. On day 8, a second therapeutically effective dose of the anti-NKp44 antibody is administered accompanied or followed by the second administration of the immunosuppressant. The skilled person will readily be able to design administration regimens which are suitable for delivery of the anti-NKp44 antibody and immunosuppressant.

In a further aspect, the invention relates to a pharmaceutical composition comprising an anti-NKp44 antibody or a fragment thereof for use in a method of treating or preventing Graft-versus-Host disease (GVHD) in a subject. The pharmaceutical composition may include further compounds, like carriers and excipients. The pharmaceutical composition may also include additional active ingredients, such as immunosuppressants as defined above.

Finally, the invention relates to a kit, comprising (a) an anti-NKp44 antibody or a fragment thereof, and (b) an immunosuppressant. The kit may also comprise instructions for administering the anti-NKp44 antibody or its fragment in combination with the immunosuppressant. In one preferred embodiment, the kit comprises, apart from the anti-NKp44 antibody or antibody fragment, a glucocorticoid such as prednisone, dexamethasone, or hydrocortisone. In another preferred embodiment, the kit comprises, apart from the anti-NKp44 antibody or antibody fragment, a calcineurin inhibitor such as tacrolimus or cyclosporine. In yet another preferred embodiment, the kit comprises, apart from the anti-NKp44 antibody or antibody fragment, an mTOR inhibitor such as sirolimus, everolimus or zotarolimus.

### EXAMPLES

The invention is illustrated by the following examples. Where cells from donors were used in the experimental studies, all donors provided written informed consent. All studies were approved by the ethical committee of the Ärztekammer Hamburg.

### Example 1: NKp44-Fc construct binding to HLA-DP molecules

With the recent description of HLA-F as a ligand for KIR3DS1, NK cell receptors for all HLA class I molecules have been identified - however, it remains unknown whether HLA class II molecules can serve as ligands for NK cell receptors. In order to assess binding of NK cell receptors to HLA class II molecules, recombinant human Fc-constructs, consisting of the extracellular domain of the respective NK cell receptor of interest and a human IgG1 Fc domain, were used in a bead-based screening assay. Briefly, the screening of HLA class II-coated beads was performed using the LABScreen Single Antigen HLA class II - Group 1 (OneLambda). Recombinant human Fc constructs (LAG-3, NKp30, NKp44, NKp46, KIR2DL3, KIR2DL1, KIR2DS1, and KIR3DS1) were purchased from R&D Systems, diluted in PBS to concentrations ranging from 1 to 100pg/mL and incubated with a mixture of 95 HLA class II coated beads for 30 minutes at room temperature. Samples were washed and incubated with F(ab')2 goat-anti-human IgG PE secondary antibody (Life Technologies) for 30 minutes at 4°C. Fc construct binding to HLA class II-coated beads was measured using Luminex xMAP technology on a Bio-Plex 200 (Bio-Rad Laboratories).

Results: In total, binding of 8 NK cell receptor Fc-constructs (NKp30, NKp44, NKp46, LAG-3, KIR2DL3, KIR2DL1, KIR3DS1, KIR2DS1) to 95 different HLA class II molecules was tested. LAG-3 has been previously described to be closely related to CD4 and to bind with high affinity to HLA class II molecules and was therefore used as a positive control. The LAG-3 Fc construct indeed interacted with all three HLA class II subtypes, HLA-DR, -DQ and -DP, respectively, while the NKp30 and the NKp46-Fc construct did not bind to any of the tested HLA class II molecules (Figure 1). Furthermore, different inhibitory (KIR2DL1, KIR2DL3) as well as activating (KIR2DS1, KIR3DS1) Fc constructs did not exhibit binding to any of the HLA class II coated beads. In contrast, the NKp44-Fc construct displayed significant binding to HLA-DP, but not to any of the HLA-DR or HLA-DQ-coated beads tested. In conclusion, these screening experiments identified a novel interaction between NKp44 and HLA-DP.

### Example 2: NKp44ζ+ Jurkat cells upregulate CD69 in response to HLA-DP

To further investigate the functional consequences of the interaction between NKp44 and HLA-DP molecules in cell-based assays, Jurkat reporter cell lines expressing an NKp44ζ or NKp46ζ construct were constructed. NKp44ζ and NKp46ζ constructs were designed by fusing the extracellular domain of the respective NCR molecule to the transmembrane domain of KIR3DL1 and the cytoplasmatic domain of the CD3ζ chain. Constructs were synthesized with GeneArt GeneSynthesis (ThermoFisher) and cloned into a lentiviral transfer plasmid encoding for a puromycin resistance. To produce lentiviral particles HEK293T were transfected using Lipofectamine 2000 (Life Technologies), a VSV-G envelope vector (pCMV-VSVg; irsiCaixa AIDS Research Institute, Badalona, Spain), an HIV-1 gag-pol packaging vector (psPAX2; NIH AIDS Reagent Program) and the transfer vector encoding the protein of interest. The Jurkat cell line (clone E6.1; ATCC) lacking β2-microglobulin (Jurkat-β2mKO cells) was previously generated (Garcia-Beltran et al. (2016), Nat. Immunol. 17, 1067-1074). Jurkat-β2mKO cells were transduced with lentiviral-like particles and selected with 1µg/mL puromycin. NKp44ζ+ and NKp46ζ+ Jurkat cells and Jurkat-β2mKO cells were cultured at 37°C and 5% CO2 in RPMI-1640 (Life Technologies) supplemented with 20% FBS (Biochrom AG). Transduced cell lines were also cultured with 1µg/mL puromycin. KIR2DL3ζ+Jurkat cells were described before in Garcia-Beltran et al. (2016), Nat. Immunol. 17, 1067-1074, and these cells were cultured under the same conditions.

Upregulation of CD69 on the surface of the NKp44ζ+ Jurkat reporter cells was used as read-out to determine ligand engagement, as described before in Garcia-Beltran et al. (2016), Nat. Immunol. 17, 1067-1074. To assess binding of NKp44 to HLA DP molecules, biotinylated HLA-DPB1*04:01 (HLA-DP401) monomers were used, as HLA-DP401 exhibited significant binding to NKp44-Fc construct in the bead-based screening assay and is currently the only HLA-DP molecules provided by the NIH tetramer facility. Furthermore, HLA-DPB1*04:01 represents one of the most frequent alleles in the Caucasian population (Al-Daccak et al. (1991), Hum. Immunol. 31, 277-285). Due to a lack of other available HLA-DP molecules HLA-DRB1*07:01 (HLA-DR7) was used as negative control in all assays. Both HLA class II monomers were loaded with the same human class II-associated invariant chain peptide (CLIP) (87-101: PVSKMRMAT-PLLMQA).

Non-tissue culture treated plates (Corning Life Sciences) were coated with 5pg/mL biotinylated HLA class II monomers (kindly provided by the NIH Tetramer Facility), 1µg/mL biotinylated anti-NKp44, 1µg/mL biotinylated anti-NKp46, 1µg/mL biotinylated anti-KIR2DL3 (Biolegend/ Miltenyi Biotec) diluted in PBS. One negative control well with only PBS was prepared for each cell line. Coated Plates were incubated at 4°C for at least 24 hours. 2.5x10⁴ Jurkat reporter cells/ well were incubated for 5h at 37°C/5% CO2 on coated plates. For blocking experiments, NKp44ζ+ Jurkat reporter cells were pre-incubated with 10pg/mL purified anti-NKp44 (Biolegend) or 10pg/mL purified isotype control antibody (Biolegend) for 30 minutes at 37°C/5% CO2 before co-incubation with coated wells, blocking antibodies remained present throughout the co-incubation. After co-incubation, cells were stained with anti-CD3 BUV737 (BD Biosciences) and anti-CD69 BV421 (Biolegend) as well as their respective NCR (anti-NKp44 PE (Biolegend), anti-NKp46 PE (BD Biosciences) or anti-KIR2DL3 PE (Miltenyi Biotec) surface molecule and fixed with 4% PFA/PBS. Level of CD69 expression was assessed using a BD LSR Fortessa (BD Biosciences).

Results: After co-incubation with HLA-DP401 CLIP molecules, but not with HLA-DR7 CLIP molecules, NKp44ζ+ Jurkat cells displayed a significant (p=0.008) upregulation of the activation marker CD69 on their surface (Figure 2A and 2B). The activation by HLA-DP401 CLIP molecules was specific to NKp44ζ+ Jurkat cells, and not observed with untransfected, NKp46ζ+ or KIR2DL3ζ+ Jurkat reporter cells. All reporter cell lines tested showed specific reporter cell activity upon co-incubation with antibodies targeting the respective NK cell receptor molecule expressed on the surface (Figure 2A and 2B). Furthermore, CD69 expression of NKp44ζ+ Jurkat cells in response to HLA-DP401 monomers was reduced when NKp44ζ+ Jurkat cells were pre-incubated with an α-NKp44 blocking antibody (median percentage of CD69+ cells: 27.3 versus 1.9) while the minor CD69 expression after co-incubation of NKp44ζ+ Jurkat cells with HLA-DR7 molecules was not negatively influenced by pre-incubation with isotype or α-NKp44 blocking antibody (median percentage of CD69+ cells: 1.2 and 2.6, respectively). In conclusion, NKp44ζ+ Jurkat reporter cell activity was specifically induced by co-incubation with HLA-DP401 monomers and this activation was blocked using an α-NKp44 blocking antibody, suggesting that the identified interaction between NKp44 and HLA-DP molecules induces a functional response.

### Example 3: Degranulation Assay

In order to test the functional response of NKp44 binding to HLA-DP401 CLIP molecules in primary human cells, NK cells were isolated from the peripheral blood of healthy donors. Human peripheral blood mononuclear cells (PBMCs) were isolated from healthy donors recruited at the university medical center Hamburg-Eppendorf using Biocoll (Biochrom AG) density centrifugation. NK cells were enriched from isolated PBMCs using magnetic labelling and negative selection with the EasySep™ human NK-cell enrichment kit (StemCell Technologies). Isolated NK cells were either directly used for degranulation assays or cultured for 7 days in RPMI-1640 supplemented with 10% FBS, 10ng/mL IL-15 and 250 U/mL IL-2 at a concentration of 2x10⁶ cells/mL.
Fresh isolated, unstimulated NK cells did not express NKp44 on the cell surface and did not degranulate upon co-incubation with α-NKp44 or HLA-DP401 CLIP -coated wells (data not shown). To induce NKp44 expression on the surface of isolated cells, NK cells were incubated with IL-2 and IL-15 for seven days. The functional response of stimulated NK cells to plate-coated α-NKp44, HLA-DR7 and HLA-DP401 CLIP was determined by the percentage of CD107a+ NK cells, as a marker for degranulation.

Therefore, non-tissue culture treated plates (Corning Life Sciences) were coated with 10µg/mL biotinylated HLA class II monomers (kindly provided by the NIH Tetramer Facility) and 1µg/mL biotinylated anti-NKp44 (Biolegend) diluted in PBS or left uncoated. Plates were incubated at 4°C for at least 24h. NK cells were resuspended at a final cell concentration of 2x10⁵ cells/mL in assay medium containing RPMI-1640 medium supplemented with 10%FBS, 250U/mL IL-2. 2x10⁴ isolated NK cells/well were distributed on coated plates or co-incubated with K562 cells at an effector:target ratio of 1:5. Brefeldin A (Sigma-Aldrich) to a final concentration of 5pg/mL and anti-CD107a (Biolegend) were added to each well and cells were incubated for 5h at 37°/5% CO2. For blocking experiments, NK cells were pre-incubated with 10pg/mL anti-NKp44 or 10pg/mL isotype control antibody (Biolegend) for 30 minutes at 37°C/5%CO2. Blocking antibody remained present during the following co-incubation. After co-incubation cells were stained with anti-CD3 BV510, anti-CD56 BV605, anti-CD16 FITC, anti-NKp44 AF647, anti-CD69 BV421 and Zombie NIR (all Biolegend). NK cells were fixed and permeabilzied using BD Cyto-fix/Cytoperm Kit (BD Biosciences) and stained intracellular with anti-TNF-a PE (Biolegend), anti-IFN-γ BUV737 (BD Biosciences) and anti-MIP-1β PerCp-eFlour 710 (eBiosciences). Samples were analyzed a using BD LSRFortessa (BD Biosciences).

Results: As can be seen in Figure 3, stimulated NK cells showed an upregulation of CD107a upon engagement with HLA-DP401 CLIP molecules (median percentage CD107a+ cells:9.38) which was significantly higher than after co-incubation with HLA-DR7 CLIP molecules (median percentage of CD107a+ cells: 2.7; p=0.03). Furthermore, the upregulation of CD107a detected after incubation with HLA-DP401 was blocked when stimulated NK cells were pre-incubated with an α-NKp44 blocking antibody (see Figure 3B) indicating that the observed functional response was mediated by the NKp44 receptor. All tested NK cells expressed CD107a after co-incubation with the NK cell sensitive tumor cell line K562 and expression of CD107a in response to K562 cells was not influenced by pre-incubation of the NK cells with an α-NKp44 blocking antibody (data not shown).

To determine whether the functional response of primary NK cells to plate-coated HLA-DP401 CLIP monomers was capable of being further increased, stimulated NK cells were sorted according to their expression of NKp44 on the cell surface. NKp44high expressing NK cells showed a slightly higher upregulation of CD107a after stimulation with plate-coated HLA-DP401 (median percentage of CD107a+ cells: 13.6) than observed in the bulk NK cell population. In contrast NKp44low expressing NK cells did not demonstrate a functional response upon co-incubation with HLA-DP401 (data not shown).

## Claims

1. Anti-NKp44 antibody or a fragment thereof for use in a method of treating or preventing Graft-versus-Host disease (GVHD) in a subject.

2. Anti-NKp44 antibody or a fragment thereof for use in a method of claim 1, wherein said anti-NKp44 antibody is a monoclonal antibody.

3. Anti-NKp44 antibody or a fragment thereof for use in a method of any of claims 1-2, wherein said anti-NKp44 antibody is a humanized antibody.

4. Anti-NKp44 antibody or a fragment thereof for use in a method of any of claims 1-3, wherein said subject is a human.

5. Anti-NKp44 antibody or a fragment thereof for use in a method of any of claims 1-4, wherein said method further comprises the administration of an immunosuppressant.

6. Anti-NKp44 antibody or a fragment thereof for use in a method of claim 5, wherein said immunosuppressant is a glucocorticoid, calcineurin inhibitor or mTOR inhibitor.

7. Anti-NKp44 antibody or a fragment thereof for use in a method of any of claims 1-6, wherein said GVDH is acute GVDH.

8. Anti-NKp44 antibody or a fragment thereof for use in a method of any of claims 1-6, wherein said GVDH is chronic GVDH.

9. Anti-NKp44 antibody or a fragment thereof for use in a method of any of claims 1-8, wherein the antibody or antibody fragment is administered at a weekly dose of 100-200 mg/m².

10. Anti-NKp44 antibody or a fragment thereof for use in a method of claims 9, wherein said weekly administration is continued for a period of at least 12 weeks.

11. Pharmaceutical composition comprising an anti-NKp44 antibody or a fragment thereof for use in a method of treating or preventing Graft-versus-Host disease (GVHD) in a subject.

12. Pharmaceutical composition for use in a method of 11, wherein said method further comprises the administration of an immunosuppressant.

13. Pharmaceutical composition for use in a method of claim 12, wherein said is a glucocorticoid, calcineurin inhibitor or mTOR inhibitor.

14. Pharmaceutical composition for use in a method of any of claims 12-13, wherein GVDH is acute or chronic GVDH.

15. Kit comprising:
a) an anti-NKp44 antibody or a fragment thereof; and
b) an immunosuppressant.
